(19) Europäisches Patentamt
European Patent Office
Office européen des brevets

(11) Publication number: 0 271 609 A1

(12) **EUROPEAN PATENT APPLICATION**

(21) Application number: 86202335.5

(22) Date of filing: 19.12.86

(51) Int. Cl.4: C07C 47/02, C07C 47/21, C07C 31/125, C07C 33/02, C12P 7/04, C12P 7/24, A23L 1/226, C11B 9/00

The title of the invention has been amended (Guidelines for Examination in the EPO, A-III, 7.3).

(43) Date of publication of application: 22.06.88 Bulletin 88/25

(84) Designated Contracting States: GB

(71) Applicant: UNILEVER PLC
Unilever House Blackfriars P.O. Box 68
London EC4P 4BQ(GB)

(72) Inventor: Harries Peter Conroy
21 Brookland Road Riseley
Bedford MK44 1EE(GB)
Inventor: Jeffcoat Roger
4 The Avenue Stanwick Wellinborough
Northants NN9 6PT(GB)
Inventor: Griffiths Evan Thomas
Maes Y Mor Bath Street Aberystwyth
Dyfed(GB)
Inventor: Trudgill Peter William
7 Maesyfelin Talybont
Dyfed SY24 5DZ(GB)

(74) Representative: Wiesenhaan, Herman, Drs. et al
Unilever N.V. Patent Division P.O.Box 137
NL-3130 AC Vlaardingen(NL)

(54) **Monoterpene aldehyde or alcohol derivatives and their use as perfumes or flavouring agents.**

(57) The invention provides a compound of the general formula:

EP 0 271 609 A1

Xerox Copy Centre

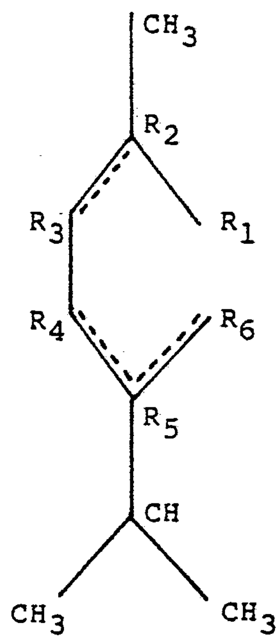

wherein $R_1$ is $CH_2OH$ or CHO,

$R_2$ and $R_5$ are CH or C,

$R_3$ and $R_4$ are $CH_2$ or CH,

$R_6$ is $CH_3$ or $CH_2$,

with the proviso that $R_1$ is not CHO when $R_3$ is CH and $R_2$ is C, $R_5$ is C, and $R_6$ is $CH_2$.

Preferably $R_1$ is CHO, $R_3$ is CH and $R_2$ is C, or $R_4$ is CH and $R_5$ is C. Also, $R_1$ can be $CH_2OH$.

The invention also provides a perfume or flavouring mixture, in which an effective amount of the above-identified compound is present.

Furthermore, the invention provides a process for preparing a compound as described above.

# NOVEL COMPOUNDS AND THEIR PREPARATION

The invention relates to novel compounds and their preparation. In particular, the invention relates to certain aldehydes and primary alcohols containing 10 carbon atoms. Specifically, the invention relates to compounds of the general formula:

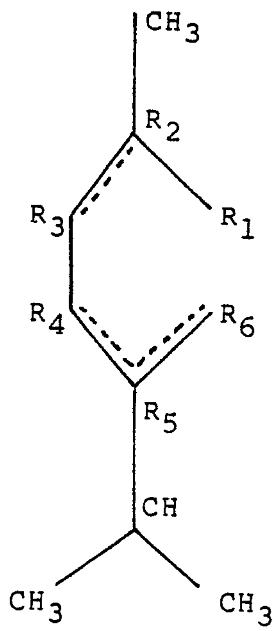

in which $R_1$ is $CH_2OH$ or CHO,

$R_2$ and $R_5$ are CH or C,

$R_3$ and $R_4$ are $CH_2$ or CH,

$R_6$ is $CH_3$ or $CH_2$,

with the proviso that $R_1$ is not CHO when $R_3$ is CH and $R_2$ is C, $R_5$ is C and $R_6$ is $CH_2$.

In this formula, the dotted line between $R_2$ and $R_3$ indicates that a carbon to carbon double bond may be present (if not, there is a single carbon to carbon bond) whereas the dotted lines between $R_5$ and $R_4$ or $R_6$ indicate that one carbon to carbon double bond between $R_5$ and $R_4$ or $R_5$ and $R_6$ may be present (if not, there is a single carbon to carbon bond).

The aldehydes/alcohols according to the present invention are valuable compounds for the perfume/flavour industry as such or as intermediates.

In an embodiment of the present invention $R_3$ is CH and $R_2$ is C in the general formula. The formula then encompasses 2-methyl-5-isopropyl hex-2-ene-l-al with optionally another double bond in the 4 position; which compounds are easily converted into the corresponding alcohols.

In particular, the invention provides 2-methyl-5-isopropyl hexa-2,4-diene-l-al, which compound is easily converted into the corresponding alcohol by borohydride reduction.

In another embodiment, the invention provides 2-methyl-5-isopropyl hexa-4-ene-l-al and the corresponding alcohol. In another embodiment, the invention provides 2-methyl-5-isopropyl hexa-4-ene-l-al and the corresponding alcohol.

The aldehydes obtained are valuable compounds for the perfumery/flavouring industry and can also be further converted into higher aldehydes e.g. by aldol condensation with aldehydes and ketones under alkaline conditions leading to aldehydes/ketones of higher chain length and which are useful per se or after reduction to the corresponding alcohol.

The invention also comprises a process for preparing a compound according to the general formula, in which a pinene (epoxide) is metabolised by the cell-free extract of a microorganism capable of growth on alpha-pinene as the sole carbon source followed by isolation of a double unsaturated compound from the incubation medium.

In accordance with the present invention, a compound according to the general formula as defined above is obtained by a microbiological process. This involves incubation of pinene epoxide with a cell-free extract of a suitable microorganism and isolation of the desired compound from the incubation medium. Sometimes a further chemical conversion follows.

A suitable microorganism is e.g. a Pseudomonas strain deposited at the National Collection of Industrial Bacteria (NCIB) deposit N° II 671 on 13th July 1981. This microorganism is described more fully in European patent specification (EP-B) 0 077 682 (Pfizer), from column I, line 52 to column 3, line 6, which is hereby incorporated as reference.

It is to be understood that the invention includes the use of other microorganisms or mutants thereof; such mutants can be obtained by various means, e.g. by irradiation with X-rays or U.V.-light or treatment with nitrogen mustards and the like mutagenic reagents. These mutants are useful provided that they have the ability to convert pinene epoxide into the desired products. This ability of any strain of microorganism for the present invention can be readily determined by cultivating the organism in the presence of pinene epoxide in accordance with the description and examples contained herein, and detecting the desired product by chromatographic or spectrophotometric methods.

Bacteria

A number of isolates, including Pseudomonas strain NCIB N° II 67I, capable of growth with alpha-pinene vapour as sole source of carbon and energy, were obtained from soil samples by standard enrichment techniques. Only one of these isolates was characterized fully, but, based on morphological characteristics on nutrient agar plates, nine apparently different strains were studied further. Analysis of the range of terpenes capable of supporting growth led to the selection of one isolate (identified as an agrobacterium) which was used primarily in this study.

Media and Culture Conditions

Bacteria were maintained on nutrient agar slants and plates.

The agrobacteria species was grown on basal salts medium containing per litre:

4 g $Na_2HPO_4$
2 g $KH_2PO_4$ pH 7.0
I g $NH_4Cl$

and 4 ml Herbert's salts (Rosenberger and Elsden, Journ. Gen. Microbiol., I960, vol. 22, p. 726) giving final concentrations (mg) per litre of growth medium as follows:

40.4 mg MgO
8.0 mg $CaCO_3$
22.4 mg $FeSO_4.7H_2O$
5.6 mg $ZnSO_4.7H_2O$
4.4 mg $MnSO_4.4H_2O$
I.0 mg $CuSO_4.5H_2O$
I.I mg $CoSO_4$ $7H_2O$
0.2 mg $H_3BO_3$

Volatile carbon sources were introduced from vapour tubes as described by Claus and Walker (Journ. Gen. Microbiol., I964, vol. 36, p. I07). Bacterial growth was monitored by following the increase in turbidity at 600 nm.

Preparation of cell-free extracts

Bacteria were harvested at the appropriate growth phase by centrifugation at I0,000 g av. for 20 min. The cell pellet was resuspended in the original volume of phosphate buffer (42 mM, pH 7.0), washed to remove residual terpenes and reharvested as described above. The final washed cell pellet was resuspended (I:I, w/v) in either phosphate buffer (pH 7.0, 42 mM) or glycine buffer (pH 9.0, 200 mM) and subsequently frozen. Cells were disrupted by a single passage of the frozen cell suspension through a Hughes Press. The lysate was incubated with I mg of crystalline DNase I (E.C. 3.I.4.5) for I5 min. at 2°C prior to the removal of undisrupted cells and large membrane fragments by centrifugation (27,000 g av., 20 min.) at 4°C. The supernatant recovered was used directly as a cell-free extract in subsequent sub-cellular enzymic studies.

A typical incubation contained cell-free extract (approx. I00µg protein/ml), alpha-pinene epoxide (approx. I mM) an glycine buffer, pH 9.0 (200 mM).

Initial recovery of the product is achieved by solvent extraction of the incubation medium. If desired, further purification can be achieved using standard techniques, for example by using chromatography or fractional distillation under reduced pressure.

Alcohols can be obtained e.g. by carrying out the incubation of 48 mM alpha-pinene oxide with cell-free extract (I5 mg/ml) in the presence of Na DH. Also passage of a suitable unsaturated aldehyde through an alumina column results in bond migration and production of other compounds.

The invention is illustrated by the following examples:

Example I

A. 2-methyl-5-isopropyl-2,5-hexadienal via 2-methyl-5-isopropyl-2,4-hexadienal II

A microorganism capable of growth on pinene as sole carbon source, Pseudomonas strain NCIB N ° II67I, was grown on pinene and converted to a cell-free extract by standard procedures. For this, bacteria were harvested at the appropriate growth phase by centrifugation at I0,000g av. for 20 min. The cell pellet was resuspended in the original volume of phosphate buffer (42 mM, pH 7.0), washed to remove residual terpenes and reharvested as described above. The final washed cell pellet was resuspended (I:I, w/v) in either phosphate buffer (pH 7.0, 42 mM) or glycine buffer (pH 9.0, 200 mM) and subsequently frozen. Cells were disrupted by a single passage of the frozen cell suspension through a Huges Press. The lysate was incubated with I mg of crystalline DNase I (E.C. 3.I.4.5.) for I5 min. at 2°C prior to the removal of undisrupted cells and large membrane fragments by centrifugation (27,000 g av., 20 min.) at 4°C. The supernatant recovered was used directly as cell-free extract in subsequent subcellular enzymic studies.

Metabolism of pinene epoxide (0.I mM/I) by this cell-free extract in the absence of added cofactors was monitored spectrophotometrically. The appearance of a peak at 235 nm highlighted the quantitative transformation of epoxide to compound I within 5 minutes. Identification of I as the known

metabolite 2-methyl-5-isopropyl-2,5-hexadienal was based on mass spectrometry (MS), carbon-13 nuclear magnetic resonance ($C_{13}$NMR), and ultraviolet spectroscopy (UV).

The electron impact MS showed a molecular ion at m/e 152-elemental composition (based on accurate mass measurement) was $C_{10}H_{16}O$. Associated peaks were observed at m/e 137 (18%), $M^+$-$CH_3$; m/e 134 (2%), M-$H_2O$; m/e 123 (15%) $M^+$-HC=O; m/e 109 (base ion), M-CH $(CH_3)_2$. In the $C_{13}$ NMR spectrum the peak at 195.3 delta was assigned to an aldehyde group (CHO). Peaks at (delta values) 152.5 (CH). 152.1 and 140.1 (C-quaternary) and 109.0 ($CH_2$) were all assigned to double bond carbons. Saturated carbon peaks were located at 34.3 (CH), 33.7 ($CH_2$), 21.5 ($CH_3$ × 2) and 9.1 ($CH_3$). The UV spectrum showed a peak at 230 nm (maximum ca. 13,000).

B. 2-methyl-5-isopropyl-2,4-hexadienal II

Passage of I through an alumina column (3% $H_2O$, activity grade II) using petroleum ether (30-40°) and diethyl ether solvents resulted in bond migration and a quantitative yield of 2-methyl-5-isopropyl-2,4-hexadienal (II). Structure elucidation of II was based on comparison of the MS, $C_{13}$ NMR and UV spectra with those of I above.

The electron impact MS of II was very similar to I with the same molecular ion (m/e 152) and elemental compostion ($C_{10}H_{16}O$). However, the base ion (m/e 109, M-CH $(CH_3)_2$) was a significantly stronger indication of an energetically more stable (conjugated) system; UV spectroscopy confirmed the higher level of conjugation (maximum 290 nm, maximum approx. 18,000). $C_{13}$NMR confirmed the loss of the peak at 109.0 delta (unsaturated $CH_2$) and the generation of a new -$CH_3$ grouping.

Example II

Preparation of 2-methyl-5-isopropyl-2,5-hexadienol (III)

Incubation of I with cell-free extract of example I in the presence of NADH was followed spectrophotometrically. After 2 hours there was an accumulation of compound III, identified as 2-methyl-5-isopropyl-2,5-hexadienol after extraction with diethyl ether. The structure was assigned on the basis of the following data.

Electron impact MS showed a molecular ion at m/e 154-elemental composition $C_{10}H_{18}O$. Other ions at m/e 139 (4%), M-$CH_3$; m/e 136 (9%) M-$H_2O$; m/e 123 (45%), M-$CH_2OH$ m/e 121 (18%), M-$CH_3/H_2O$; m/e 111 (14%) M-CH$(CH_3)_2$. $C_{13}$NMR gave peaks at (delta values) 154.5 and 135.9 (C-quaternaries), 124.2 (CH) and 107.2 ($CH_2$) all assigned to unsaturated carbon atoms. Saturated carbon peaks assigned were at 68.9 ($CH_2O$), 33.9 (CH), 32.6 ($CH_2$), 21.7 ($CH_3$ × 2) and 13.6 ($CH_3$). The UV spectrum showed no peaks in the 225-400 nm range

## Claims

1. A compound of the general formula:

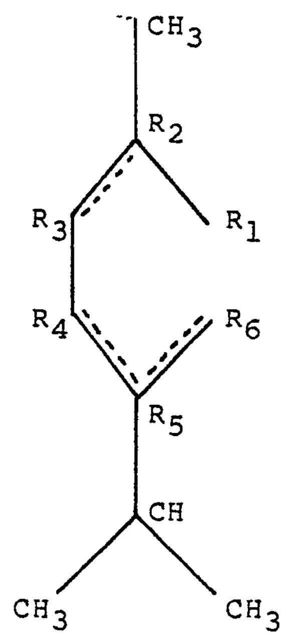

characterized in that $R_1$ is $CH_2OH$ or CHO,
$R_2$ and $R_5$ are CH or C,
$R_3$ and $R_4$ are $CH_2$ or CH,
$R_6$ is $CH_3$ or $CH_2$,
with the proviso that $R_1$ is not CHO when $R_3$ is CH and $R_2$ is C, $R_5$ is C, and $R_6$ is $CH_2$.

2. A compound according to claim 1, characterized in that $R_1$ is CHO.

3. A compound according to claim 2, characterized in that $R_3$ is CH and $R_2$ is C.

4. A compound according to claim 2 or 3, characterized in that $R_4$ is CH and $R_5$ is C.

5. A compound according to claim 1, characterized in that $R_1$ is $CH_2OH$.

6. A compound according to claim 5, characterized in that $R_3$ is CH and $R_2$ is C.

7. A compound according to claims 5 and 6, characterized in that $R_4$ is CH and $R_5$ is C.

8. A compound according to claims 5 and 6, characterized in that $R_5$ is C and $R_6$ is $CH_2$.

9. A perfume or flavouring mixture, characterized in that it contains an effective amount of a compound according to any of the claims 1-8.

I0. A process for preparing a compound according to claim I, characterized in that pinene (epoxide) is metabolized by the cell-free extract of a microorganism capable of growth on pinene (epoxide) as a carbon source followed by isolation of the compound from the incubation medium.

II. A process according to claim I0, characterized in that the cell-free extract is one obtained from a Pseudomonas strain as identified by deposit N° II 67I of the NCIB.

European Patent Office

# EUROPEAN SEARCH REPORT

Application number

EP 86 20 2335

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | CHEMICAL ABSTRACTS, vol. 97, no. 11, 13th September 1982, page 722, abstract no. 91741j, Columbus, Ohio, US; & JP-A-82 72 931 (TAKASAGO PERFUMERY CO., LTD.) 07-05-1982 * Abstract * | 1-9 | C 07 C 47/02<br>C 07 C 47/21<br>C 07 C 31/125<br>C 07 C 33/02<br>C 12 P 7/04<br>C 12 P 7/24<br>A 23 L 1/226<br>C 11 B 9/00 |
| X | JOURNAL OF THE AMERICAN CHEMICAL SOCIETY, vol. 85, no. 7, 5th April 1963, pages 955-958, Gaston, PA, US; E.P. BLANCHARD, Jr. et al.: "A new method for the conversion of glycidic esters to aldehydes and ketones" * Whole article, particularly page 956, formula XIII * | 1-8 | |
| A | CHEMICAL ABSTRACTS, vol. 88, no. 15, 10th April 1978, page 236, abstract no. 101326h, Columbus, Ohio, US; N.J. TUDROSZEN et al.: "Alpha-pinene metabolism by Pseudomonas putida", & BIOCHEM. J. 1977, 168(2), 315-18 | 1,10, 11 | TECHNICAL FIELDS SEARCHED (Int. Cl.4)<br>C 07 C 47/00<br>C 07 C 31/00<br>C 07 C 33/00 |

The present search report has been drawn up for all claims

| Place of search | Date of completion of the search | Examiner |
|---|---|---|
| THE HAGUE | 14-08-1987 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document